# EUROPÄISCHE PATENTANMELDUNG

(11) **EP 4 378 492 A1**
(43) Veröffentlichungstag der Anmeldung: **05.06.2024**
(21) Anmeldenummer: 22210849.0
(22) Anmeldetag: 01.12.2022
(51) Int. Cl.: A61L 29/08, C08L 69/00

(54) **MEDIZINPRODUKT MIT HOHER INTRALIPIDBESTÄNDIGKEIT AUS POLYCARBONATMATERIAL**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Beschrieben ist die Verwendung von funktionalisiertem Siloxan-Polymer zur Verbesserung der Intralipidbeständigkeit von aromatischem Polycarbonat, was insbesondere bei medizintechnischen Produkten, die Elemente umfassen, welche dafür bestimmt sind, bei der bestimmungsgemäßen Verwendung der medizintechnischen Produkte in Kontakt mit Intralipid-Lösung zu kommen, von Relevanz ist.

## Beschreibung

Die Erfindung betrifft medizintechnische Produkte mit Elementen aus thermoplastischem Kunststoff, welche dafür vorgesehen sind, während der bestimmungsgemäßen Verwendung des medizintechnischen Produktes mit Intralipid in Kontakt zu kommen.

Aromatisches Polycarbonat ist ein attraktives Material, das auch in einigen Bereichen der Medizintechnik zum Einsatz kommt. Je nach gefordertem Eigenschaftsprofil sind aber bisher längst nicht für alle gewünschten Anwendungsfelder geeignete Polycarbonat-basierte Zusammensetzungen identifiziert. Es wäre jedoch im Zuge der Kreislaufwirtschaft wünschenswert, Polycarbonat auch bei weiteren als den bisher bekannten Produkten, beispielsweise bei Autoinjektoren, Injektionshilfen und Dialysatoren, verwenden zu können, um möglichst wenige verschiedene Materialien einzusetzen und diese damit möglicherweise zusammen recyceln zu können.

Bei Medizinprodukten wie Dreiwegehähnen für die enterale oder auch für die parenterale Ernährung ist die Intralipidbeständigkeit von großer Bedeutung. Intralipid ist eine Lipidemulsion auf Basis von Sojabohnenöl. In der künstlichen Ernährung mittels intravenöser Anwendung dient es als Fettquelle. Daneben weist es auch weitere vorteilhafte Eigenschaften auf, z.B. verbessert es die Bioverfügbarkeit von Antikrebsmedikamenten auf Platinbasis. Es scheint außerdem die Behandlung von Autoimmunerkrankungen zu unterstützen, indem es die Aktivität der natürlichen Killerzellen senkt und außerdem die Behandlung bei Auftreten wiederholter Fehlgeburten zu fördern.

Bisher wird als geeignetes Material POM (Polyoxymethylen) für solche Medizinprodukte, die bei ihrer bestimmungsgemäßen Anwendung mit Intralipiden in Kontakt gebracht werden, eingesetzt. Polycarbonat hingegen ist für solche Anwendungen bisher kein attraktives Material. Die Chemikalienbeständigkeit von aromatischem Polycarbonat als solchem ist begrenzt und sofern ein Kontakt des Materials mit Intralipiden erfolgen soll, ist selbst herkömmliches als chemikalienbeständig geltendes Spezialpolycarbonat nicht für entsprechende Anwendungen geeignet.

Um die Chemikalienbeständigkeit bei Polycarbonat zu erhöhen, erhöht man üblicherweise das Molekulargewicht. Dieses ist allerdings nur bis zu einem bestimmten Grad möglich, bis zu Molekulargewichten M_{w} von etwa 31 000 bis 34 000 g/mol, bestimmt mittels GPC, kalibriert gegen Bisphenol A-Polycarbonat Standards mit Dichlormethan als Elutionsmittel. Mit der Erhöhung des Molekulargewichts lässt die Fließfähigkeit und somit die Verarbeitbarkeit zunehmend nach.

Aufgabe war es daher, ein Intralipid-beständiges Polycarbonatmaterial bereitzustellen, das die Intralipidbeständigkeit auf anderem Wege als durch Erhöhung des Molekulargewichtes erhält, um entsprechend in Medizinprodukten, die für den direkten Kontakt mit Intralipiden bestimmt sind, eingesetzt zu werden.

Überraschend hat sich gezeigt, dass auf aromatischem Polycarbonat basierende Zusammensetzungen eine deutlich verbesserte Intralipidbeständigkeit aufweisen, wenn sie funktionalisiertes Siloxan-Polymer, aufgebaut aus Siloxan-Einheiten, insbesondere Polydimethylsiloxan, enthalten. Dieser positive Einfluss zeigt sich auch bei vergleichsweise geringen Molekulargewichten des Polycarbonats, die das Material sonst als nicht geeignet für entsprechende Medizinprodukte deklassifiziert hätten, d.h. Polycarbonat mit einer Schmelzevolumenfließrate MVR von 15 bis 35 cm³/(10 min), bestimmt gemäß ISO 1133:2012-03, bei einer Prüftemperatur 300°C und 1,2 kg Belastung. Entsprechende Ausführungsformen mit Polycarbonaten mit eher niedrigem Molekulargewicht sind besonders attraktiv, da ohne ansonsten erforderliche weitergehende Additivierung zur Fließverbesserung Zusammensetzungen eingesetzt werden können, die sich sehr gut verarbeiten lassen.

Gegenstand der Erfindung ist somit ein
medizintechnisches Produkt oder Teil eines medizintechnischen Produktes,
wobei das medizintechnische Produkt oder Teil eines medizintechnischen Produktes ein Element, bestehend aus einer thermoplastischen Zusammensetzung, welche die folgenden Komponenten enthält, umfasst:
   A) mindestens 75 Gew.-% aromatisches Polycarbonat und
   B) 0,2 bis 1,5 Gew.-% funktionalisiertes Siloxan-Polymer, das nur aus Siloxan-Einheiten als Monomereinheiten aufgebaut ist,
wobei sich die Mengenangaben auf das Gesamtgewicht der thermoplastischen Zusammensetzung beziehen und
wobei das Element dazu bestimmt ist, während der bestimmungsgemäßen Verwendung des medizintechnischen Produktes in Kontakt mit Intralipid zu stehen.

Gegenstand der Erfindung ist außerdem die Verwendung von funktionalisiertem Siloxan-Polymer, das nur aus Siloxan-Einheiten als Monomereinheiten aufgebaut ist, zur Verbesserung der Intralipidbeständigkeit von auf aromatischem Polycarbonat basierenden Zusammensetzungen.

Es versteht sich, dass ein "medizintechnisches Produkt, das ein Element bestehend aus einer definierten Zusammensetzung umfasst" bzw. "Teil eines medizintechnischen Produktes, das ein Element, bestehend aus einer definierten Zusammensetzung umfasst", auch solche medizintechnischen Produkte bzw. Teile von medizintechnischen Produkten umfasst, die "nur" aus der definierten Zusammensetzung bestehen. Ebenso versteht es sich, dass das "Element" insbesondere ein Formteil aus der thermoplastischen Zusammensetzung ist und dass "bestehend aus" hierbei nicht ausschließt, dass weitere Schichten oder anknüpfende Elemente an dem Element angebracht sind. Es ist es denkbar, dass das Element, bestehend aus der thermoplastischen Zusammensetzung beispielsweise eine oder mehrere Beschichtungsschichten aufweist, die aber nicht über seine gesamte Oberfläche reichen. "Element" kann auch lediglich ein Teilbereich eines Formteils sein, erhalten etwa durch Mehrkomponentenspritzguss, wobei eben dieses Element, dieser Teilbereich bei der bestimmungsgemäßen Verwendung des medizintechnischen Produktes in direkten Kontakt mit Intralipid gebracht wird. Ebenso kann das Element die Beschichtungsschicht oder eine Coextrusionsschicht eines Formteils aus einem anderen thermoplastischen Material sein, die mindestens in dem Bereich vorhanden ist, in dem das Formteil bei seiner bestimmungsgemäßen Verwendung in Kontakt mit Intralipid steht.

Aus Kostenersparnisgründen und da die erfindungsgemäß eingesetzte thermoplastische Zusammensetzung bereits eine sehr hohe Intralipidbeständigkeit aufweist, weist das Formteil aus der thermoplastischen Zusammensetzung bevorzugt keine weiteren Schichten auf.

Intralipid, welches bei der bestimmungsgemäßen Verwendung des medizintechnischen Produktes in unmittelbarem, also direktem Kontakt mit dem Element aus der thermoplastischen Zusammensetzung steht, ist vorzugsweise solches, welches für die parenterale Ernährung vorgesehen ist, bevorzugt eines, das mittelkettige Triglyceride enthält, wobei "mittelkettig" bevorzugt C₆ bis C₁₂ bedeutet. Entsprechende Intralipide enthalten typischerweise eine Mischung aus Sojabohnenöl, Glyerin und Phospholipiden aus Eiern. Ein solches typisches Intralipid ist beispielsweise eine Mischung, enthaltend Aminosäuren wie Alanin, Arginin, Glycin, Histidin, Isoleucin, Leucin, Lysinacetat, Methionin, Phenylalanin, Prolin, Serin, Taurin, Threonin, Tryptophan, Tyrosin, Valin, aber auch raffiniertes Sojaöl, mittelkettige Triglyceride, raffiniertes Olivenöl, Omega-3-Säuren-reiches Fischöl, Glucose (Kohlenhydrate), Stickstoff, Acetat (Anteil aus der Aminosäurelösung), Phosphat (Anteil aus der Lipidemulsion), Glycerol, Eilecithin, Alpha-Tocopherol (Ph.Eur.), Natriumhydroxid, Natriumoleat, Essigsäure 99 %, Salzsäure 10 %, Wasser. Bevorzugt ist das Intralipid SmofKabiven Electrolytfrei Emulsion der Fresenius Kabi AG.

Bevorzugt ist das medizintechnische Produkt oder Teil eines medizintechnischen Produktes ein Schlauchverbinder, ein Dreiwegehahn, ein Luer-Konnektor, ein Manifold, eine Tropfkammer, ein IV-Katheter.

### Komponente A

Komponente A der erfindungsgemäßen Zusammensetzungen sind aromatische Polycarbonate.

Aromatische Polycarbonate im Sinne der vorliegenden Erfindung sind sowohl Homopolycarbonate als auch Copolycarbonate und/oder Polyestercarbonate; die Polycarbonate können in bekannter Weise linear oder verzweigt sein. Erfindungsgemäß können auch Mischungen von Polycarbonaten verwendet werden.

Die Schmelzevolumenfließrate, MVR des eingesetzten aromatischen Polycarbonats, bestimmt gemäß ISO 1133:2012-03, bei einer Prüftemperatur 300°C und 1,2 kg Belastung, beträgt bevorzugt 6 bis 35 cm³/(10 min), weiter bevorzugt 15 cm³/(10 min) bis 35 cm³/(10 min), noch weiter bevorzugt 16 bis 30 cm³/(10 min), besonders bevorzugt 16 bis 20 cm³/(10 min), ganz besonders bevorzugt 18 bis 20 cm³/(10 min). Sofern die erfindungsgemäß verwendeten thermoplastischen Zusammensetzungen eine Mischung verschiedener aromatischer Polycarbonate enthalten, so sind etwaige Vorzugsbereiche als Angabe für die Gesamtmischung aromatischer Polycarbonate zu verstehen.

Ein Teil, bis zu 80 Mol-%, vorzugsweise von 20 Mol-% bis zu 50 Mol-%, der Carbonat-Gruppen in den erfindungsgemäß eingesetzten Polycarbonaten können durch aromatische DicarbonsäureesterGruppen ersetzt sein. Derartige Polycarbonate, die sowohl Säurereste der Kohlensäure als auch Säurereste von aromatischen Dicarbonsäuren in die Molekülkette eingebaut enthalten, werden als aromatische Polyestercarbonate bezeichnet. Sie werden im Rahmen der vorliegenden Erfindung unter dem Oberbegriff der thermoplastischen, aromatischen Polycarbonate subsumiert.

Einzelheiten der Herstellung von Polycarbonaten sind in vielen Patentschriften seit etwa 40 Jahren niedergelegt. Beispielhaft sei hier auf Schnell, "Chemistry and Physics of Polycarbonates", Polymer Reviews, Volume 9, Interscience Publishers, New York, London, Sydney 1964, auf D. Freitag, U. Grigo, P.R. Müller, H. Nouvertne, BAYER AG, "Polycarbonates" in Encyclopedia of Polymer Science and Engineering, Volume 11, Second Edition, 1988, Seiten 648-718 und schließlich auf U. Grigo, K. Kirchner und P.R. Müller "Polycarbonate" in Becker/Braun, Kunststoff-Handbuch, Band 3/1, Polycarbonate, Polyacetale, Polyester, Celluloseester, Carl Hanser Verlag München, Wien 1992, Seiten 117 bis 299 verwiesen.

Die Herstellung aromatischer Polycarbonate erfolgt z.B. durch Umsetzung von Dihydroxyarylverbindungen mit Kohlensäurehalogeniden, vorzugsweise Phosgen, und/oder mit aromatischen Dicarbonsäuredihalogeniden, vorzugsweise Benzoldicarbonsäuredihalogeniden, nach dem Phasengrenzflächenverfahren, gegebenenfalls unter Verwendung von Kettenabbrechern und gegebenenfalls unter Verwendung von trifunktionellen oder mehr als trifunktionellen Verzweigern. Ebenso ist eine Herstellung über ein Schmelzepolymerisationsverfahren durch Umsetzung von Dihydroxyarylverbindungen mit beispielsweise Diphenylcarbonat möglich.

Für die Herstellung der Polyestercarbonate wird ein Teil der Kohlensäurederivate durch aromatische Dicarbonsäuren oder Derivate der Dicarbonsäuren ersetzt, und zwar je nach Maßgabe der in den aromatischen Polycarbonaten zu ersetzenden Carbonatstruktureinheiten durch aromatische Dicarbonsäureesterstruktureinheiten.

Für die Herstellung von Polycarbonaten geeignete Dihydroxyarylverbindungen sind solche der Formel (1)

HO-Z-OH (1),

in welcher
- Z: ein aromatischer Rest mit 6 bis 30 C-Atomen ist, der einen oder mehrere aromatische Kerne enthalten kann, substituiert sein kann und aliphatische oder cycloaliphatische Reste bzw. Alkylaryle oder Heteroatome als Brückenglieder enthalten kann.

Bevorzugt steht Z in Formel (1) für einen Rest der Formel (2) 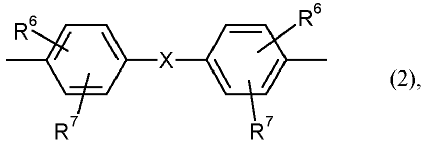 in der
- R⁶ und R⁷: unabhängig voneinander für H, C₁- bis C₁₈-Alkyl-, C₁- bis C₁₈-Alkoxy, Halogen wie Cl oder Br oder für jeweils gegebenenfalls substituiertes Aryl- oder Aralkyl, bevorzugt für H oder C₁- bis C₁₂-Alkyl, besonders bevorzugt für H oder C₁- bis C₈-Alkyl und ganz besonders bevorzugt für H oder Methyl stehen, und
- X: für eine Einfachbindung, -SO₂-, -CO-, -O-, -S-, C₁- bis C₆-Alkylen, C₂- bis C₅-Alkyliden oder C₅-bis C₆-Cycloalkyliden, welches mit C₁- bis C₆-Alkyl, vorzugsweise Methyl oder Ethyl, substituiert sein kann, ferner für C₆- bis C₁₂-Arylen, welches gegebenenfalls mit weiteren Heteroatome enthaltenden aromatischen Ringen kondensiert sein kann, steht.

Bevorzugt steht X für eine Einfachbindung, C₁- bis C₅-Alkylen, C₂- bis C₅-Alkyliden, C₅- bis C₆-Cycloalkyliden, -O-, -SO-, -CO-, -S-, -SO₂-
oder für einen Rest der Formel (3)

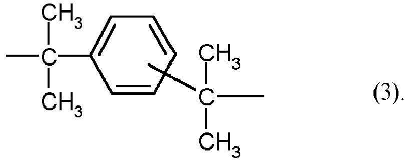

Beispiele für Dihydroxyarylverbindungen sind: Dihydroxybenzole, Dihydroxydiphenyle, Bis-(hydroxyphenyl)-alkane, Bis-(hydroxyphenyl)-cycloalkane, Bis-(hydroxyphenyl)-aryle, Bis-(hydroxyphenyl)-ether, Bis-(hydroxyphenyl)-ketone, Bis-(hydroxyphenyl)-sulfide, Bis-(hydroxyphenyl)-sulfone, Bis-(hydroxyphenyl)-sulfoxide, 1,1'-Bis-(hydroxyphenyl)-diisopropylbenzole sowie deren kernalkylierte und kernhalogenierte Verbindungen.

Für die Herstellung der Polycarbonate geeignete Dihydroxyarylverbindungen sind beispielsweise Hydrochinon, Resorcin, Dihydroxydiphenyle, Bis-(hydroxyphenyl)-alkane, Bis-(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α-α'-Bis-(hydroxyphenyl)-diisopropylbenzole, Phthalimidine, abgeleitet von Isatin- oder Phenolphthaleinderivaten, sowie deren kernalkylierte, kernarylierte und kernhalogenierte Verbindungen.

Bevorzugte Dihydroxyarylverbindungen sind 4,4'-Dihydroxydiphenyl, 2,2-Bis-(4-hydroxyphenyl)-propan (Bisphenol A), 2,4-Bis-(4-hydroxyphenyl)-2-methylbutan, 1,1-Bis-(4-hydroxyphenyl)-p-diisopropylbenzol, 2,2-Bis-(3-methyl-4-hydroxyphenyl)-propan, Dimethyl-Bisphenol A, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,1-Bis-(3,5-dimethyl-4-hydroxyphenyl)-p-diisopropylbenzol und 1,1-Bis-(4-hydroxyphenyl)-3,3,5-trimethylcyclohexan, sowie Dihydroxyarylverbindungen (I) bis (III) 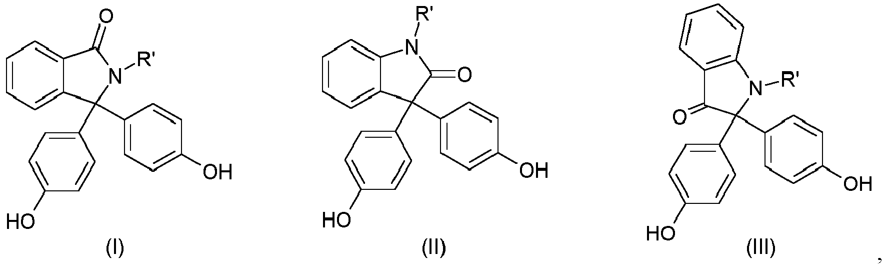 in denen R' jeweils für einen C₁- bis C₄-Alkylrest, Aralkylrest oder Arylrest, bevorzugt für einen Methylrest oder Phenylrest, ganz besonders bevorzugt für einen Methylrest, steht.

Besonders bevorzugte Dihydroxyarylverbindungen sind 2,2-Bis-(4-hydroxyphenyl)-propan (Bisphenol A), 2,2-Bis-(3,5-dimethyl-4-hydroxyphenyl)-propan, 1,1-Bis-(4-hydroxyphenyl)-cyclohexan, 1,1-Bis-(4-hydroxyphenyl)-3,3,5-trimethylcyclohexan, 4,4'-Dihydroxydiphenyl und Dimethyl-Bisphenol A sowie die Bisphenole der Formeln (I), (II) und (III).

Diese und weitere geeignete Dihydroxyarylverbindungen sind z.B. in US 3 028 635 A, US 2 999 835 A, US 3 148 172 A, US 2 991 273 A, US 3 271 367 A, US 4 982 014 A und US 2 999 846 A, in DE 1 570 703 A, DE 2063 050 A, DE 2 036 052 A, DE 2 211 956 A und DE 3 832 396 A, in FR 1 561 518 A, in der Monographie "H. Schnell, Chemistry and Physics of Polycarbonates, Interscience Publishers, New York 1964" sowie in JP 62039/1986 A, JP 62040/1986 A und JP 105550/1986 A beschrieben.

Im Fall der Homopolycarbonate wird nur eine Dihydroxyarylverbindung eingesetzt, im Fall der Copolycarbonate werden zwei oder mehr Dihydroxyarylverbindungen eingesetzt.

Geeignete Kohlensäurederivate sind beispielsweise Phosgen oder Diphenylcarbonat.

Geeignete Kettenabbrecher, die bei der Herstellung der Polycarbonate eingesetzt werden können, sind Monophenole. Geeignete Monophenole sind beispielsweise Phenol selbst, Alkylphenole wie Kresole, p-tert.-Butylphenol, Cumylphenol sowie deren Mischungen.

Bevorzugte Kettenabbrecher sind die Phenole, welche ein- oder mehrfach mit C₁- bis C₃₀-Alkylresten, linear oder verzweigt, bevorzugt unsubstituiert, oder mit tert-Butyl substituiert sind. Besonders bevorzugte Kettenabbrecher sind Phenol, Cumylphenol und/oder p-tert-Butylphenol.

Die Menge an einzusetzendem Kettenabbrecher beträgt bevorzugt 0,1 bis 5 Mol-%, bezogen auf Mole an jeweils eingesetzten Dihydroxyarylverbindungen. Die Zugabe der Kettenabbrecher kann vor, während oder nach der Umsetzung mit einem Kohlensäurederivat erfolgen.

Geeignete Verzweiger sind die in der Polycarbonatchemie bekannten tri- oder mehr als trifunktionellen Verbindungen, insbesondere solche mit drei oder mehr als drei phenolischen OH-Gruppen.

Geeignete Verzweiger sind beispielsweise 1,3,5-Tri-(4-hydroxyphenyl)-benzol, 1,1,1-Tri-(4-hydroxyphenyl)-ethan, Tri-(4-hydroxyphenyl)-phenylmethan, 2,4-Bis-(4-hydroxyphenylisopropyl)-phenol, 2,6-Bis-(2-hydroxy-5'-methyl-benzyl)-4-methylphenol, 2-(4-Hydroxyphenyl)-2-(2,4-dihydroxyphenyl)-propan, Tetra-(4-hydroxyphenyl)-methan, Tetra-(4-(4-hydroxyphenylisopropyl)-phenoxy)-methan und 1,4-Bis-((4',4"-dihydroxytriphenyl)-methyl)-benzol und 3,3-Bis-(3-methyl-4-hydroxyphenyl)-2-oxo-2,3-dihydroindol.

Die Menge der gegebenenfalls einzusetzenden Verzweiger beträgt bevorzugt 0,05 Mol-% bis 2,00 Mol-%, bezogen auf Mole an jeweils eingesetzten Dihydroxyarylverbindungen.

Die Verzweiger können entweder mit den Dihydroxyarylverbindungen und den Kettenabbrechern in der wässrig alkalischen Phase vorgelegt werden oder in einem organischen Lösungsmittel gelöst vor der Phosgenierung zugegeben werden. Im Fall des Umesterungsverfahrens werden die Verzweiger zusammen mit den Dihydroxyarylverbindungen eingesetzt.

Besonders bevorzugte Polycarbonate sind das Homopolycarbonat auf Basis von Bisphenol A, das Homopolycarbonat auf Basis von 1,1-Bis-(4-hydroxyphenyl)-3,3,5-trimethylcyclohexan und die Copolycarbonate auf Basis der beiden Monomere Bisphenol A und 1,1-Bis-(4-hydroxyphenyl)-3,3,5-trimethylcyclohexan beziehungsweise der beiden Monomere Bisphenol A und 4,4'-Dihydroxydiphenyl, sowie von den Dihydroxyarylverbindungen der Formeln (I), (II) und/oder (III) 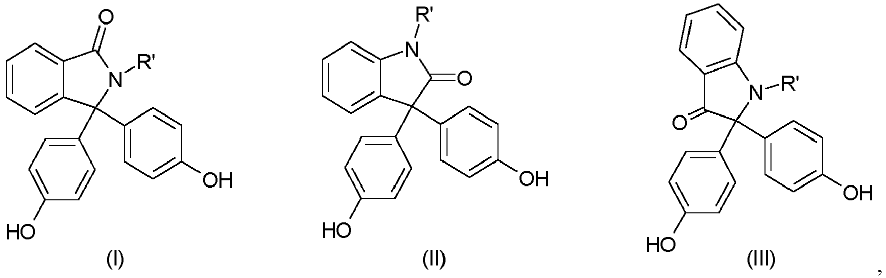 in denen R' jeweils für C₁- bis C₄-Alkyl, Aralkyl oder Aryl, bevorzugt für Methyl oder Phenyl, ganz besonders bevorzugt für Methyl, steht,
abgeleitete Homo- oder Copolycarbonate, insbesondere mit Bisphenol A. Ganz besonders bevorzugt umfasst das aromatische Polycarbonat ein Bisphenol A-basiertes Homopolycarbonat. Äußerst bevorzugt ist das aromatische Polycarbonat Bisphenol A-basiertes Homopolycarbonat.

Der Gesamtanteil der Monomereinheiten beruhend auf den Formeln (I), (II), (III), 4,4'-Dihydroxydiphenyl und/oder Bisphenol TMC im Copolycarbonat beträgt vorzugsweise 0,1 - 88 mol%, besonders bevorzugt 1 - 86 mol-%, ganz besonders bevorzugt 5 - 84 mol-% und insbesondere 10 - 82 mol-% (bezogen auf die Summe der Mole eingesetzter Dihydroxyarylverbindungen).

Die relative Lösungsviskosität der Copolycarbonate, bestimmt nach ISO 1628-4:1999, liegt bevorzugt im Bereich von = 1,15 - 1,35.

Die verwendeten Dihydroxyarylverbindungen, wie auch alle anderen der Synthese zugesetzten Chemikalien und Hilfsstoffe, können mit den aus ihrer eigenen Synthese, Handhabung und Lagerung stammenden Verunreinigungen kontaminiert sein. Es ist jedoch wünschenswert, mit möglichst reinen Rohstoffen zu arbeiten.

Bevorzugt sind auch Copolycarbonate, zu deren Herstellung Diphenole der allgemeinen Formel (4a) eingesetzt wurden: 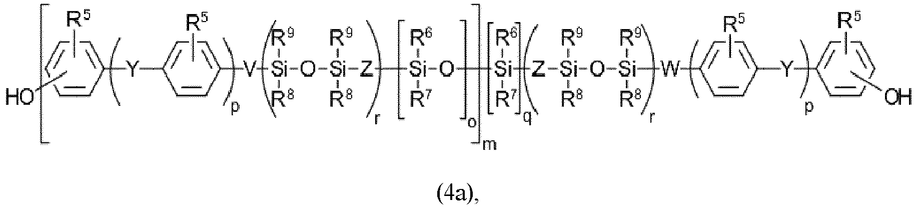 wobei
R⁵ für Wasserstoff oder C₁- bis C₄- Alkyl, C₁- bis C₃-Alkoxy, vorzugsweise für Wasserstoff; Methoxy oder Methyl, steht,
R⁶, R⁷, R⁸ und R⁹ jeweils unabhängig voneinander für C₁- bis C₄-Alkyl oder C₆- bis C₁₂-Aryl, vorzugsweise für Methyl oder Phenyl stehen,
Y für eine Einfachbindung, SO₂-, -S-, -CO-, -O-, C₁- bis C₆-Alkylen, C₂- bis C₅-Alkyliden, C₆- bis C₁₂-Arylen, welches gegebenenfalls mit weiteren Heteroatome enthaltenden aromatischen Ringen kondensiert sein kann oder für einen C₅- bis C₆-Cycloalkylidenrest, der ein- oder mehrfach mit C₁bis C₄-Alkyl substituiert sein kann, bevorzugt für eine Einfachbindung, -O-, Isopropyliden oder für einen C₅- bis C₆-Cycloalkylidenrest, der ein- oder mehrfach mit C₁- bis C₄-Alkyl substituiert sein kann, steht,
V für Sauerstoff, C₂- bis C₆-Alkylen oder C₃- bis C₆-Alkyliden, bevorzugt für Sauerstoff oder C₃-Alkylen,
p, q und r jeweils unabhängig für 0 oder 1 stehen,
wenn q = 0 ist, W für eine Einfachbindung steht, wenn q = 1 und r = 0 ist, W für Sauerstoff, C₂- bis C₆-Alkylen oder C₃- bis C₆-Alkyliden, bevorzugt für Sauerstoff oder C₃-Alkylen steht,
wenn q = 1 und r = 1 ist, W und V jeweils unabhängig für C₂- bis C₆-Alkylen oder C₃- bis C₆-Alkyliden, bevorzugt C₃-Alkylen steht,
Z für ein C₁- bis C₆-Alkylen, bevorzugt C₂-Alkylen steht,
o für eine durchschnittliche Anzahl von Wiederholungseinheiten von 10 bis 500, bevorzugt 10 bis 100 steht, und
m für eine durchschnittliche Anzahl von Wiederholungseinheiten von 1 bis 10, bevorzugt 1 bis 6, weiter bevorzugt 1,5 bis 5 steht. Ebenso ist es möglich, dass Diphenole verwendet werden, in denen zwei oder mehrere Siloxanblöcke der allgemeinen Formel (4a) über Terephthalsäure und/oder Isophthalsäure miteinander unter Ausbildung von Estergruppen verknüpft sind.

Insbesondere bevorzugt sind (Poly)Siloxane der Formeln (5) und (6) 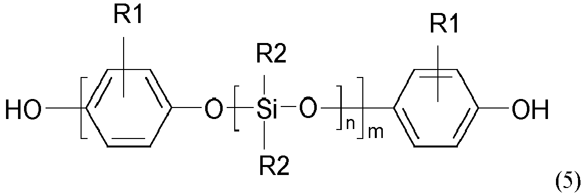 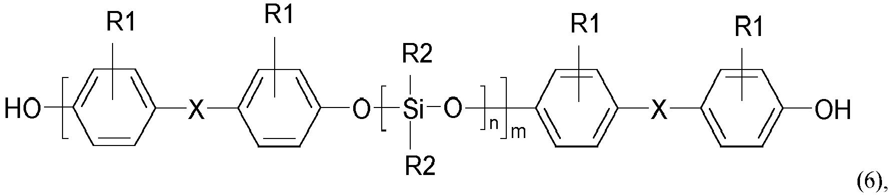 wobei R1 für Wasserstoff, C₁- bis C₄-Alkyl, vorzugsweise für Wasserstoff oder Methyl steht und insbesondere bevorzugt für Wasserstoff,
R2 unabhängig voneinander für Aryl oder Alkyl, bevorzugt für Methyl,
X für eine Einfachbindung, -SO₂-, -CO-, -O-, -S-, C₁- bis C₆-Alkylen, C₂- bis Cs-Alkyliden oder für C₆- bis C₁₂-Arylen, welches gegebenenfalls mit weiteren Heteroatome enthaltenden aromatischen Ringen kondensiert sein kann, steht,
X bevorzugt für eine Einfachbindung, C₁- bis C₅-Alkylen, C₂- bis Cs-Alkyliden, C₅- bis C₁₂-Cycloalkyliden, -O-, -SO- -CO-, -S-, -SO₂-, besonders bevorzugt steht X für eine Einfachbindung, Isopropyliden, C₅- bis C₁₂-Cycloalkyliden oder Sauerstoff, und ganz besonders bevorzugt für Isopropyliden steht,
n eine durchschnittliche Zahl von 10 bis 400, bevorzugt 10 und 100, insbesondere bevorzugt 15 bis 50 bedeutet und
m für eine durchschnittliche Zahl von 1 bis 10, bevorzugt von 1 bis 6 und insbesondere bevorzugt von 1,5 bis 5 steht.

Ebenso bevorzugt kann der Siloxanblock von folgender Struktur abgeleitet sein 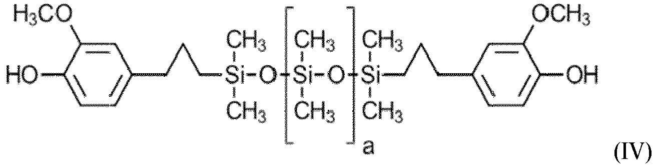 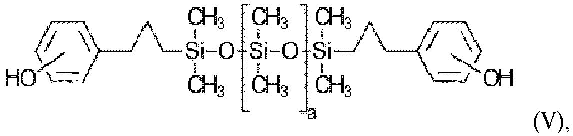 bevorzugt (Va) 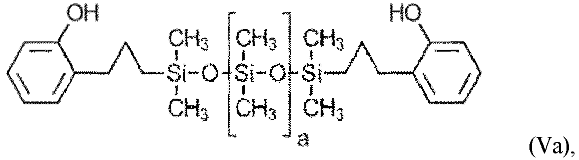 oder 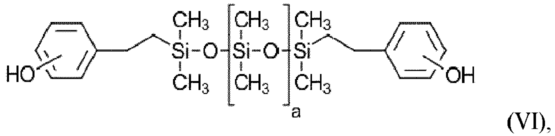 wobei a in Formel (IV), (V) und (VI) für eine durchschnittliche Zahl von 10 bis 400, bevorzugt 10 bis 100 und besonders bevorzugt für 15 bis 50 steht.

Dabei ist es ebenso bevorzugt, dass mindestens zwei gleiche oder verschiedene der Siloxanblöcke der allgemeinen Formeln (IV), (V) oder (VI) über Terephthalsäure und/oder Isophthalsäure miteinander unter Ausbildung von Estergruppen verknüpft sind.

Ebenso ist es bevorzugt, wenn in der Formel (4a) p = 0 ist, V für C₃-Alkylen steht, r = 1 ist, Z für C₂-Alkylen steht, R⁸ und R⁹ für Methyl stehen, q = 1 ist, W für C₃-Alkylen steht, m = 1 ist, R⁵ für Wasserstoff oder C₁- bis C₄-Alkyl, vorzugsweise für Wasserstoff oder Methyl, steht, R⁶ und R⁷ jeweils unabhängig voneinander für C₁- bis C₄-Alkyl, vorzugsweise für Methyl stehen und o für 10 bis 500 steht.

Copolycarbonate mit Monomereinheiten der Formel (4a) und insbesondere auch deren Herstellung sind in der WO 2015/052106 A2 beschrieben.

Copolycarbonate mit Monomereinheiten der Formel (IV) und insbesondere auch deren Herstellung sind in der WO 2015/052106 A2 beschrieben.

Für die Herstellung der Polyestercarbonate geeignete aromatische Dicarbonsäuren sind beispielsweise Orthophthalsäure, Terephthalsäure, Isophthalsäure, tert-Butylisophthalsäure, 3,3'-Diphenyldicarbonsäure, 4,4'-Diphenyldicarbonsäure, 4,4-Benzophenondicarbonsäure, 3,4'-Benzophenondicarbonsäure, 4,4'-Diphenyletherdicarbonsäure, 4,4'-Diphenylsulfondicarbonsäure, 2,2-Bis-(4-carboxyphenyl)-propan, Trimethyl-3-phenylindan-4,5'-dicarbonsäure.

Von den aromatischen Dicarbonsäuren werden besonders bevorzugt die Terephthalsäure und/oder Isophthalsäure eingesetzt.

Derivate der Dicarbonsäuren sind die Dicarbonsäuredihalogenide und die Dicarbonsäuredialkylester, insbesondere die Dicarbonsäuredichloride und die Dicarbonsäuredimethylester.

Der Ersatz der Carbonatgruppen durch die aromatischen Dicarbonsäureestergruppen erfolgt im Wesentlichen stöchiometrisch und auch quantitativ, so dass das molare Verhältnis der Reaktionspartner sich auch im fertigen Polyestercarbonat wiederfindet. Der Einbau der aromatischen Dicarbonsäureestergruppen kann sowohl statistisch als auch blockweise erfolgen.

Die erfindungsgemäßen Zusammensetzungen enthalten mindestens 75 Gew.-%, bevorzugt mindestens 85 Gew.-%, weiter bevorzugt mindestens 90 Gew.-%, besonders bevorzugt mindestens 95 Gew.-%, äußerst bevorzugt mindestens 97,5 Gew.-% aromatisches Polycarbonat, basieren also auf aromatischem Polycarbonat.

### Komponente B

Komponente B ist funktionalisiertes Siloxan-Polymer, das nur aus Siloxan-Einheiten als Monomereinheiten aufgebaut ist, dessen Gruppe der Monomereinheiten somit aus Siloxan-Einheiten besteht. Bei Komponente B kann es sich um ein einziges Siloxan-Polymer oder eine Mischung von zwei oder mehr Siloxan-Polymeren handeln. Bevorzugt weist das Siloxan-Polymer - wenn es eine Mischung verschiedener Siloxan-Polymere ist, in seiner Gesamtheit - ein gewichtsmittleres Molekulargewicht M_{w} von > 500 000 g/mol, weiter bevorzugt von > 750 000 g/mol, besonders bevorzugt von > 1 000 000 g/mol, bestimmt mittels GPC in Tetrahydrofuran (THF), kalibriert gegen Polystyrol-Standards, auf, ist also ein UHMW (ultra high molecular weight) Siloxan-Polymer.

"Nur aus... aufgebaut" bedeutet vorliegend, dass diesen Polymeren keine weiteren Monomerbausteine zugrunde liegen. Es sind lineare oder verzweigte Polymere oder Copolymere, wobei bevorzugt die organischen Gruppen der Siloxan-Einheiten unabhängig voneinander aus Methyl oder Phenyl-Gruppen ausgewählt sind. Geeignete Siloxan-Polymere gemäß Komponente B schließen Polydimethylsiloxan-Homopolymere und Copolymere, die aus Dimethylsiloxan- und Methylphenylsiloxan-Einheiten aufgebaut sind, Copolymere, die aus Dimethylsiloxan- und Diphenylsiloxan-Einheiten aufgebaut sind, Copolymere, die aus Diphenylsiloxan-Einheiten und Methylphenylsiloxan-Einheiten aufgebaut sind und Homopolymere von Methylphenylsiloxan-Einheiten ein. Mischungen aus zwei oder mehr dieser Polymere oder Copolymere können auch für Blends aus dem zuvor beschriebenen höher- und/oder niedrigermolekularen Siloxan-Polymer eingesetzt werden. Besonders bevorzugt ist Komponente B mindestens ein Polymer, ausgewählt aus der Gruppe, bestehend aus Polydimethylsiloxan-Homopolymeren und Copolymeren, die aus Dimethylsiloxan- und Methylphenylsiloxan-Einheiten aufgebaut sind, Copolymeren, die aus Dimethylsiloxan- und Diphenylsiloxan-Einheiten aufgebaut sind, Copolymeren, die aus Diphenylsiloxan-Einheiten und Methylphenylsiloxan-Einheiten aufgebaut sind, Homopolymeren von Methylphenylsiloxan-Einheiten oder Mischungen der vorgenannten Polymere.

Bei dem Siloxan-Polymer gemäß Komponente B handelt es sich um funktionalisiertes Siloxan-Polymer. "Funktionalisiert" bedeutet in diesem Zusammenhang, dass das Siloxan-Polymer in seiner Gesamtheit eine oder mehrere reaktive Gruppen aufweist. Unter "reaktiven Gruppen" werden erfindungsgemäß an dieser Stelle solche Gruppen aufgefasst, die grundsätzlich die Stoffeigenschaften und das Reaktionsverhalten der Verbindung maßgeblich bestimmen. Solche reaktiven Gruppen sind beispielsweise Hydroxyl-, Methyl-, Fluoro-, Carboxyl-, Stickstoff-haltige Gruppen -wie Amin- - oder Alkenyl-Gruppen. Besonders bevorzugt umfasst das Siloxan-Polymer Hydroxylgruppen. "In seiner Gesamtheit" bedeutet hierbei, dass nicht jedes Siloxan-Polymer der Mischung entsprechende Gruppen aufweisen muss, aber dass Siloxan-Polymer enthalten sein muss, welches reaktive Gruppen umfasst. Bevorzugt weist das gesamte Siloxan-Polymer gemäß Komponente B eine oder mehrere Vertreter reaktiver Gruppen auf, insbesondere mindestens Hydroxylgruppen.

Die reaktiven Gruppen sind am Ende des Moleküls und/oder entlang der Polymerkette angeordnet, vorzugsweise sind sie an den Kettenenden angeordnet. Ganz besonders bevorzugt sind als reaktive Gruppen an den Kettenenden angeordnete Hydroxylgruppen vorhanden, äußerst bevorzugt in Form von Diorganohydroxysiloxygruppen, beispielweise Dimethylhydroxysiloxy-, Diphenylhydroxysiloxy- und/oder Methylphenylhydroxysiloxygruppen.

Sofern die reaktiven Gruppen nur entlang der Polymerkette angeordnet sind, sind die terminalen Gruppen des Siloxan-Polymers nicht reaktiv und typischerweise eine Di- oder Triorganosiloxyspezies, z.B. Dimethylvinylsiloxy- oder Trimethylsiloxygruppen.

Besonders bevorzugt umfasst Komponente B Hydroxylgruppen-terminiertes Polydimethylsiloxan, ganz besonders bevorzugt ist Komponente B Hydroxylgruppen-terminiertes Polydimethylsiloxan.

Äußerst bevorzugt ist das Siloxan-Polymer gemäß Komponente B funktionalisiertes lineares Polydimethylsiloxan, enthaltend bis zu 50 mol-% Methylreste, bevorzugt ist dabei das Siloxan-Polymer Polydimethylsiloxan-Homopolymer mit Dimethylhydroxysiloxyendgruppen.

### Komponente C

Die erfindungsgemäß zum Einsatz kommenden thermoplastischen Zusammensetzungen können außerdem ein oder mehrere Additive enthalten, die hier als Komponente C bezeichnet sind. "Können" bedeutet, dass nicht zwingend ein weiteres Additiv enthalten sein muss; die Menge an Komponente C kann somit auch 0 Gew.-% betragen. Die Menge an weiteren Additiven beträgt bevorzugt bis zu 15 Gew.-%, weiter bevorzugt bis zu 10 Gew.-%, besonders bevorzugt bis zu 5 Gew.-%, ganz besonders bevorzugt bis zu 1,0 Gew.-%.

Solche weiteren Additive, wie sie üblicherweise Polycarbonaten zugesetzt werden, sind insbesondere Thermostabilisatoren, Bestrahlungsstabilisatoren, Flammschutzmittel, Antioxidantien, Entformungsmittel, Antitropfmittel, etwa Polytetrafluorethylen (Teflon) oder SAN-gekapseltes PTFE (z.B. Blendex 449), UV-Absorber, IR-Absorber, Schlagzähmodifikatoren, optische Aufheller, Füllstoffe, z. B. Talk, Silikate oder Quarz, Lichtstreumittel, Hydrolysestabilisatoren, Umesterungsstabilisatoren, Verträglichkeitsvermittler, organische Farbmittel, organische Pigmente, anorganische Pigmente und/oder Additive zur Lasermarkierung, insbesondere in den für Polycarbonat-basierte Zusammensetzungen üblichen Mengen. Derartige Additive sind beispielsweise in EP-A 0 839 623, WO-A 96/15102, EP-A 0 500 496 oder im "Plastics Additives Handbook", Hans Zweifel, 5th Edition 2000, Hanser Verlag, München beschrieben. Diese Additive können einzeln oder auch im Gemisch zugesetzt werden.

Es versteht sich, dass nur solche Additive und nur in solchen Mengen zugesetzt werden dürfen, wenn sie sich nicht signifikant negativ auf den erfindungsgemäßen Effekt der Verbesserung der Intralipidbeständigkeit auswirken.

Bevorzugt enthalten die thermoplastischen Zusammensetzungen mindestens ein oder mehrere Entformungsmittel, ein oder mehrere Thermostabilisatoren und/oder Antioxidantien sowie optional Farbmittel als weitere Additive.

Geeignete Entformungsmittel sind insbesondere solche auf Basis eines Fettsäureesters, noch weiter bevorzugt auf Basis eines Stearinsäureesters, insbesondere bevorzugt auf Pentaerythritbasis. Besonders bevorzugt werden Pentaerythrittetrastearat (PETS) und/oder Glycerinmonostearat (GMS) eingesetzt. Sofern ein oder mehrere Entformungsmittel eingesetzt werden, beträgt die Menge bevorzugt bis 1,0 Gew.-% (einschließlich), weiter bevorzugt 0,01 bis 0,7 Gew.-%, besonders bevorzugt 0,02 bis 0,60 Gew.-%, jeweils bezogen auf die Gesamtzusammensetzung.

Sofern Thermostabilisator eingesetzt wird, beträgt die Menge bevorzugt bis 0,20 Gew.-%, weiter bevorzugt 0,01 bis 0,10 Gew.-%, noch weiter bevorzugt 0,01 bis 0,05 Gew.-%, besonders bevorzugt 0,015 bis 0,040 Gew.-%, bezogen auf die Gesamtzusammensetzung.

Als Thermostabilisatoren sind insbesondere Phosphor-basierte Stabilisatoren, ausgewählt aus der Gruppe der Phosphate, Phosphite, Phosphonite, Phosphine und deren Mischungen, geeignet. Beispiele sind Triphenylphosphit, Diphenylalkylphosphit, Phenyldialkylphosphit, Tris(nonylphenyl)phosphit, Trilaurylphosphit, Trioctadecylphosphit, Distearylpentaerythritoldiphosphit, Tris(2,4-di-tert-butylphenyl)phosphit (Irgafos^{®} 168), Diisodecylpentaerythritoldiphosphit, Bis(2,4-di-tert-butylphenyl)pentaerythritoldiphosphit, Bis(2,4-di-cumylphenyl)pentaerythritoldiphosphit (Doverphos^{®} S-9228), Bis(2,6-di-tert-butyl-4-methylphenyl)penta-erythritol-diphosphit, Diisodecyloxypentaerythritoldiphosphit, Bis(2,4-di-tert-butyl-6-methylphenyl)-pentaerythritoldiphosphit, Bis(2,4,6-tris(tert-butylphenyl)pentaerythritoldiphosphit, Tristearylsorbitoltriphosphit, Tetrakis(2,4-di-tert-butylphenyl)-4,4'-biphenylendiphosphonit, 6-Isooctyloxy-2,4,8,10-tetra-tert-butyl-12H-dibenz[d,g]-1,3,2-dioxaphosphocin, Bis(2,4-di-tert-butyl-6-methylphenyl)methylphosphit, Bis(2,4-di-tert-butyl-6-methylphenyl)ethylphosphit, 6-Fluoro-2,4,8,10-tetra-tert-butyl-12-methyl-dibenz[d,g]-1,3,2-dioxaphosphocin, 2,2',2"-Nitrilo-[triethyltris(3,3',5,5'-tetra-tert-butyl-1,1'-biphenyl-2,2'-diyl)phosphit], 2-Ethylhexyl(3,3',5,5'-tetra-tert-butyl-1,1'-biphenyl-2,2'-diyl)phosphit, 5-Butyl-5-ethyl-2-(2,4,6-tri-tert-butylphenoxy)-1,3,2-dioxaphosphiran, Bis(2,6-di-ter-butyl-4-methylphenyl)pentaerythritol-diphosphit, Triphenylphosphin (TPP), Trialkylphenylphosphin, Bisdiphenylphosphino-ethan oder ein Trinaphthylphosphin. Sie werden allein oder im Gemisch, z. B. Irganox^{®} B900 (Gemisch aus Irgafos^{®} 168 und Irganox^{®} 1076 im Verhältnis 4:1) oder Doverphos^{®} S-9228 mit Irganox^{®} B900 bzw. Irganox^{®} 1076, eingesetzt. Insbesondere bevorzugt werden Triphenylphosphin (TPP), Irgafos^{®} 168 oder Tris(nonylphenyl)phosphit oder deren Mischungen eingesetzt.

Ferner können phenolische Antioxidantien wie alkylierte Monophenole, alkylierte Thioalkylphenole, Hydrochinone und alkylierte Hydrochinone eingesetzt werden. Besonders bevorzugt werden Irganox^{®} 1010 (Pentaerythrit-3-(4-hydroxy-3,5-di-tert-butylphenyl)propionat; CAS: 6683-19-8) und Irganox 1076^{®} (Octadecyl-3-(3,5-di-tert-butyl-4-hydroxyphenyl)-propionat) eingesetzt, bevorzugt in Mengen von 0,05 - 0,5 Gew-%.

Ferner können Sulfonsäureester oder Alkylphosphate, z. B. Mono-, Di- und/oder Trihexylphosphat, Triisoctylphosphat und/oder Trinonylphosphat, als Umesterungsinhibitoren zugegeben werden. Bevorzugt, sofern Umesterungsstabilisator enthalten ist, wird als Alkylphosphat Triisooctylphosphat (Tris-2-ethyl-hexyl-phosphat) verwendet. Es können auch Mischungen aus verschiedenen Mono-, Di- und Trialkylphosphaten verwendet werden. Triisooctylphosphat wird bevorzugt in Mengen von 0,003 Gew.-% bis 0,05 Gew.-%, weiter bevorzugt 0,005 Gew.-% bis 0,04 Gew.-% und besonders bevorzugt von 0,01 Gew.-% bis 0,03 Gew.-%, bezogen auf die Gesamtzusammensetzung, eingesetzt.

Die Zusammensetzungen, die für die erfindungsgemäßen medizintechnischen Produkte eingesetzt werden, werden üblicherweise wie folgt hergestellt:
Zur Einarbeitung von Additiven wird das aromatische Polycarbonat gemäß Komponente A, auf dem die thermoplastische Zusammensetzung basiert, ggf. als Thermoplastgemisch, bevorzugt in Form von Pulvern, Granulaten oder von Gemischen aus Pulvern und Granulaten eingesetzt.

Außer den Additiven können die erfindungsgemäß zum Einsatz kommenden Zusammensetzungen optional, ggf. zusätzlich zu den Additiven, auch einen oder mehrere Blendpartner umfassen. Geeignete Blendpartner sind beispielsweise ABS, Polyester wie PBT oder PET, PMMA oder deren Mischungen.

Komponente B wie auch die weiteren Bestandteile, d.h. insbesondere die weiteren Additive, werden mit gängigen Einarbeitungsverfahren durch Zusammenführung, Vermischen und Homogenisieren der einzelnen Bestandteile in entsprechende Zusammensetzungen überführt, wobei insbesondere die Homogenisierung bevorzugt in der Schmelze unter Einwirkung von Scherkräften stattfindet. Gegebenenfalls erfolgt das Zusammenführen und Vermischen vor der Schmelzehomogenisierung unter Verwendung von Pulvervormischungen.

Es können auch Vormischungen aus Granulaten oder Granulaten und Pulvern mit den einzelnen Komponenten verwendet werden.

Es können auch Vormischungen verwendet werden, die aus Lösungen der Mischungskomponenten in geeigneten Lösungsmitteln hergestellt worden sind, wobei gegebenenfalls in Lösung homogenisiert wird und das Lösungsmittel anschließend entfernt wird.

Insbesondere können hierbei die Komponenten der Zusammensetzungen durch bekannte Verfahren oder als Masterbatch in das thermoplastische Polymer, insbesondere in das aromatische Polycarbonat, ggf. in das aromatische Polycarbonat mit Blendpartner, eingebracht werden. Bevorzugt wird mindestens Komponente B als Masterbatch in aromatischem Polycarbonat, weiter bevorzugt in Bisphenol A-basiertem Homopolycarbonat, in die thermoplastische Zusammensetzung eingebracht.

Die Verwendung von Masterbatchen, hier also auf Basis von aromatischem Polycarbonat, ist zum Einbringen der jeweiligen Komponenten, einzeln oder in Mischung, bevorzugt. Dieses gilt insbesondere auch für Komponente B, das Siloxan-Polymer. Beim Einbringen mittels Masterbatch enthält dieser bevorzugt 0,5 bis 60 Gew.-%, weiter bevorzugt 20 bis 55 Gew.-%, besonders bevorzugt 45 bis 52 Gew.-% Siloxan-Polymer, bezogen auf das Gesamtgewicht des Masterbatches. Durch das Einbringen als Masterbatch wird üblicherweise eine homogene Verteilung des Additivs möglich.

Die erfindungsgemäß zum Einsatz kommende Zusammensetzung kann in üblichen Vorrichtungen wie Schneckenextrudern (zum Beispiel Zweischneckenextruder, ZSK), Knetern, Brabender- oder Banbury-Mühlen zusammengeführt, vermischt, homogenisiert und anschließend extrudiert werden. Nach der Extrusion kann das Extrudat abgekühlt und zerkleinert werden. Es können auch einzelne Komponenten vorgemischt werden und dann können die restlichen Ausgangsstoffe einzeln und/oder ebenfalls gemischt hinzugegeben werden.

Die Zusammenführung und Durchmischung einer Vormischung in der Schmelze kann auch in der Plastifiziereinheit einer Spritzgussmaschine erfolgen. Hierbei wird die Schmelze im anschließenden Schritt direkt in einen Formkörper überführt.

Die Zusammensetzungen können auf üblichen Maschinen, beispielsweise auf Extrudern oder Spritzgussmaschinen, zu beliebigen Formkörpern in üblicher Weise verarbeitet werden.

Erfindungsgemäß bevorzugt ist ein
medizintechnisches Produkt oder Teil eines medizintechnischen Produktes,
wobei das medizintechnische Produkt oder Teil eines medizintechnischen Produktes ein Element, bestehend aus einer thermoplastischen Zusammensetzung, welche die folgenden Komponenten enthält, umfasst:
   A) mindestens 90 Gew.-% aromatisches Polycarbonat und
   B) 0,2 bis 1,5 Gew.-% funktionalisiertes Siloxan-Polymer, das nur aus Siloxan-Einheiten als Monomereinheiten aufgebaut ist, wobei das funktionalisierte Siloxan-Polymer ein gewichtsmittleres Molekulargewicht M_{w}, bestimmt mittels GPC in Tetrahydrofuran (THF), kalibriert gegen Polystyrol-Standards, von > 500 000 g/mol aufweist, Dimethylsiloxan-Einheiten und Hydroxylgruppen aufweist,
      wobei sich die Mengenangaben auf das Gesamtgewicht der thermoplastischen Zusammensetzung beziehen und
      wobei das Element dazu bestimmt ist, während der bestimmungsgemäßen Verwendung des medizintechnischen Produktes in Kontakt mit Intralipid zu stehen.
Erfindungsgemäß weiter bevorzugt ist ein
medizintechnisches Produkt oder Teil eines medizintechnischen Produktes,
wobei das medizintechnische Produkt oder Teil eines medizintechnischen Produktes ein Element, bestehend aus einer thermoplastischen Zusammensetzung, welche aus den folgenden Komponenten besteht, umfasst:
   A) mindestens 90 Gew.-% aromatisches Polycarbonat und
   B) 0,2 bis 1,5 Gew.-% funktionalisiertes Siloxan-Polymer, das nur aus Siloxan-Einheiten als Monomereinheiten aufgebaut ist,
      wobei das funktionalisierte Siloxan-Polymer vorzugsweise ein gewichtsmittleres Molekulargewicht M_{w}, bestimmt mittels GPC in Tetrahydrofuran (THF), kalibriert gegen Polystyrol-Standards, von > 500 000 g/mol sowie Dimethylsiloxan-Einheiten sowie Hydroxylgruppen aufweist,
   C) optional einem oder mehreren weiteren Additiven, ausgewählt aus der Gruppe, bestehend aus Thermostabilisatoren, Flammschutzmitteln, Antioxidantien, Entformungsmitteln, Antitropfmitteln, UV-Absorbern, IR-Absorbern, Schlagzähmodifikatoren, optischen Aufhellern, Füllstoffen, Lichtstreumitteln, Hydrolysestabilisatoren, Umesterungsstabilisatoren, Verträglichkeitsvermittlern, organischen Farbmitteln, organischen Pigmenten, anorganischen Pigmenten und/oder Additiven zur Lasermarkierung,
wobei sich die Mengenangaben auf das Gesamtgewicht der thermoplastischen Zusammensetzung beziehen und
wobei das Element dazu bestimmt ist, während der bestimmungsgemäßen Verwendung des medizintechnischen Produktes in Kontakt mit Intralipid zu stehen.

Erfindungsgemäß besonders bevorzugt ist ein
medizintechnisches Produkt oder Teil eines medizintechnischen Produktes,
wobei das medizintechnische Produkt oder Teil eines medizintechnischen Produktes ein Element, bestehend aus einer thermoplastischen Zusammensetzung, welche aus den folgenden Komponenten besteht, umfasst:
   A) mindestens 90 Gew.-% aromatisches Polycarbonat und
   B) 0,2 bis 1,5 Gew.-% funktionalisiertes Siloxan-Polymer, das nur aus Siloxan-Einheiten als Monomereinheiten aufgebaut ist,
      wobei das funktionalisierte Siloxan-Polymer vorzugsweise ein gewichtsmittleres Molekulargewicht M_{w}, bestimmt mittels GPC in Tetrahydrofuran (THF), kalibriert gegen Polystyrol-Standards, von > 500 000 g/mol sowie Dimethylsiloxan-Einheiten sowie Hydroxylgruppen aufweist,
   C) optional einem oder mehreren weiteren Additiven, ausgewählt aus der Gruppe, bestehend aus Entformungsmitteln, Thermostabilisatoren, Antioxidantien, Farbmitteln, Pigmenten,
wobei sich die Mengenangaben auf das Gesamtgewicht der thermoplastischen Zusammensetzung beziehen und
wobei das Element dazu bestimmt ist, während der bestimmungsgemäßen Verwendung des medizintechnischen Produktes in Kontakt mit Intralipid zu stehen.

Erfindungsgemäß ganz besonders bevorzugt ist ein
medizintechnisches Produkt oder Teil eines medizintechnischen Produktes,
wobei das medizintechnische Produkt oder Teil eines medizintechnischen Produktes ein Element, bestehend aus einer thermoplastischen Zusammensetzung, welche aus den folgenden Komponenten besteht, umfasst:
   A) mindestens 90 Gew.-% aromatisches Polycarbonat, wobei das aromatische Polycarbonat vorzugsweise Bisphenol A-Homopolycarbonat enthält, besonders bevorzugt Bisphenol A-Homopolycarbonat ist, und
   B) 0,2 bis 1,5 Gew.-%, insbesondere 0,5 bis 1 Gew.-% funktionalisiertes Siloxan-Polymer, das nur aus Siloxan-Einheiten als Monomereinheiten aufgebaut ist,
      wobei das funktionalisierte Siloxan-Polymer vorzugsweise ein gewichtsmittleres Molekulargewicht M_{w}, bestimmt mittels GPC in Tetrahydrofuran (THF), kalibriert gegen Polystyrol-Standards, von > 500 000 g/mol sowie Dimethylsiloxan-Einheiten sowie Hydroxylgruppen aufweist,
   C) optional einem oder mehreren weiteren Additiven, ausgewählt aus der Gruppe, bestehend aus Entformungsmitteln, Thermostabilisatoren, Antioxidantien, Farbmitteln, Pigmenten,
wobei sich die Mengenangaben auf das Gesamtgewicht der thermoplastischen Zusammensetzung beziehen und
wobei das Element dazu bestimmt ist, während der bestimmungsgemäßen Verwendung des medizintechnischen Produktes in Kontakt mit Intralipid zu stehen.

Äußerst bevorzugt ist ein
medizintechnisches Produkt oder Teil eines medizintechnischen Produktes,
wobei das medizintechnische Produkt oder Teil eines medizintechnischen Produktes ein Element, bestehend aus einer thermoplastischen Zusammensetzung, welche aus den folgenden Komponenten besteht, umfasst:
   A) mindestens 90 Gew.-% aromatisches Polycarbonat, wobei das aromatische Polycarbonat Bisphenol A-Homopolycarbonat ist, eine Schmelzevolumenfließrate MVR von 16 bis 20 cm³/(10 min), bestimmt gemäß ISO 1133:2012-03, bei einer Prüftemperatur 300°C und 1,2 kg Belastung, aufweist, und
   B) 0,2 bis 1,5 Gew.-%, insbesondere 0,5 bis 1 Gew.-% funktionalisiertes Siloxan-Polymer, das nur aus Siloxan-Einheiten als Monomereinheiten aufgebaut ist,
      wobei das funktionalisierte Siloxan-Polymer ein gewichtsmittleres Molekulargewicht M_{w}, bestimmt mittels GPC in Tetrahydrofuran (THF), kalibriert gegen Polystyrol-Standards, von > 500 000 g/mol sowie Dimethylsiloxan-Einheiten sowie Hydroxylgruppen aufweist,
   C) optional einem oder mehreren weiteren Additiven, ausgewählt aus der Gruppe, bestehend aus Entformungsmitteln, Thermostabilisatoren, Antioxidantien, Farbmitteln, Pigmenten,
wobei sich die Mengenangaben auf das Gesamtgewicht der thermoplastischen Zusammensetzung beziehen und
wobei das Element dazu bestimmt ist, während der bestimmungsgemäßen Verwendung des medizintechnischen Produktes in Kontakt mit Intralipid zu stehen.

In den vorstehend beschriebenen bevorzugten, weiter bevorzugten etc. Ausführungsformen ist das funktionalisierte Siloxan-Polymer besonders bevorzugt ein Dimethylsiloxan-Homopolymer, mindestens funktionalisiert mit Hydroxylgruppen, ganz besonders bevorzugt ausschließlich mit Hydroxylgruppen funktionalisiert.

Die für das erfindungsgemäße medizintechnische Produkt bzw. Teil eines medizintechnischen Produktes vorstehend als bevorzugt, weiter bevorzugt etc. beschriebenen Ausführungsformen gelten - soweit anwendbar - auch für die erfindungsgemäße Verwendung der Komponente B, also die Verwendung zur Verbesserung der Intralipidbeständigkeit von auf aromatischem Polycarbonat basierenden Zusammensetzungen, welche vorzugsweise mindestens 75 Gew.-% aromatisches Polycarbonat, weiter bevorzugt mindestens 90 Gew.-% aromatisches Polycarbonat enthalten.

Die nachfolgenden Beispiele sollen die Erfindung illustrieren, ohne sie jedoch einzuschränken.

### Beispiele

### 1. Komponenten

**Komponente A-1:** Lineares Homopolycarbonat auf Basis von Bisphenol-A mit einer SchmelzeVolumenfließrate (MVR) von 19 cm³/(10 min) (gemäß ISO 1133:2012-03, bei einer Prüftemperatur von 300°C und einer Prüflast von 1,2 kg).

**Komponente A-2:** Lineares Homopolycarbonat auf Basis von Bisphenol-A mit einer SchmelzeVolumenfließrate (MVR) von 6 cm³/(10 min) (gemäß ISO 1133:2012-03, bei einer Prüftemperatur von 300°C und einer Prüflast von 1,2 kg).

**Komponente A-3:** Lineares Homopolycarbonat auf Basis von Bisphenol-A mit einer SchmelzeVolumenfließrate (MVR) von 5 cm³/(10 min) (gemäß ISO 1133:2012-03, bei einer Prüftemperatur von 300°C und einer Prüflast von 1,2 kg).

**Komponente B*-1:** Pelletisierte Zusammensetzung enthaltend 50 Gew.-% mit Hydroxylendgruppen funktionalisiertes UHMW Siloxan-Polymer (Polydimethylsiloxan), welches in aromatischem Polycarbonat dispergiert ist. **Komponente B,** funktionalisiertes Siloxan-Polymer, ist somit in den Beispielen 2 und 3 zu 0,5 Gew.-% bzw. 1 Gew.-%, bezogen auf die Gesamtzusammensetzung, enthalten.

**Komponente C-1:** Entformungsmittel Pentaerythrittetrastearat von Emery Oleochemicals.

**Komponente C-2:** Additivpaket aus Multranol 3600 DHP (Alpha, omega-bis(tetrahydro-2H-pyran-2-yl)-poly[oxy(methyl-1,2-ethanediyl)], Polyetherpolyol. Mₙ = 2,000 g/mol) sowie zwei Anthrachinonfarbmitteln.

**Eingesetztes Intralipid:** SmofKabiven Electrolytfrei Emulsion der Fresenius Kabi AG. Wirkstoffe: 1000 ml enthalten: Raffiniertes Sojaöl (Ph.Eur.) 11,4 g, mittelkettige Triglyceride 11,4 g, raffiniertes Olivenöl 9,5 g, Omega-3-Säuren-reiches Fischöl 5,7 g, Glucose (als Glucose-Monohydrat (Ph.Eur.)) 127 g, Alanin 7,1 g, Arginin 6,1 g, Glycin 5,6 g, Histidin 1,5 g, Isoleucin 2,5 g, Leucin 3,8 g, Lysinacetat 3,4 g, Methionin 2,2 g, Phenylalanin 2,6 g, Prolin 5,7 g, Serin 3,3 g, Taurin 0,5 g, Threonin 2,2 g, Tryptophan 1,0 g, Tyrosin 0,20 g, Valin 3,1 g, entsprechend Aminosäuren 51 g, Stickstoff 8 g, Kohlenhydrate (Glucose wasserfrei) 127 g, Lipide 38 g, Acetat (Anteil aus der Aminosäurelösung) 74,5 mmol, Phosphat (Anteil aus der Lipidemulsion) 2,8 mmol. Gesamtenergie ca. 1100 kcal (4,6 MJ), Nichteiweißenergie ca. 900 kcal (3,8 MJ). Osmolalität ca. 1600 mosm/kg Wasser, Osmolarität ca. 1300 mosm/l, pH-Wert (nach Mischen) ca. 5,6. Sonstige Bestandteile: Glycerol, Eilecithin, Alpha-Tocopherol (Ph.Eur.), Natriumhydroxid, Natriumoleat, Essigsäure 99 %, Salzsäure 10 %, Wasser für Injektionszwecke. Für die intravenöse Infusion für die parenterale Ernährung.

### Durchführung

Die in den folgenden Beispielen beschriebenen Polycarbonatzusammensetzungen wurden auf einem Extruder ZE 25 der Fa. Berstorff mit einem Durchsatz von 10 kg/Std. durch Compoundierung hergestellt. Die Schmelzetemperatur betrug 275 °C.

Als Maß für die Chemikalienbeständigkeit dient die umgebungsbedingte Spannungsrissbildung (ESC). Die ESC wurde mittels Biegestreifenverfahren bei Raumtemperatur bestimmt. Dabei wurde ein bei 280°C Massetemperatur und 80°C Werkzeugtemperatur abgespritztes Prüfkörper der Abmessungen 80 mm x 10 mm x 4 mm mittels einer Biegeschablone mit einer externen Randfaserdehnung (Rfd.) von 1,4% beaufschlagt. Unmittelbar nachdem der Probekörper aufgespannt war, wurde der Probekörper mit dem Prüfmedium (SmofKabiven Elektrolyfrei Emulsion) in Kontakt gebracht. Hierzu wurde das Prüfmedium tropfenweise auf gewebeartiges Papier aufgetragen, welches dann auf die Probe (mittig, in der Zugzone des Probekörpers) gelegt wurde. Die aufgespannten Probekörper wurden für einen Tag bzw. vier Tage mit Prüfmedium gelagert. Anschließend wurden die Probekörper von der Biegeschablone gelöst und einer Sichtprüfung unterzogen, um die Oberflächenbeschaffenheit der Prüfkörper nach den in Tabelle 1 beschriebenen Kriterien zu bestimmen:

**Tabelle 1: Kriterien für die Bewertung der Chemikalienbeständigkeit**

| Eigenschaft | Versagenskriterium | Kurzbezeichnung |
|---|---|---|
| Oberflächenbeschaffenheit (bewertet durch Sichtprüfung) | Risse oder Haarrisse an den Kanten der gedehnten Oberfläche | A1 |
| | Risse oder Haarrisse an der gedehnten Oberfläche | A2 |
| | keine Veränderung | kV |

**Tabelle 2: Durchgeführte Versuche und Ergebnisse**

| **Komponenten** | | **1 (Vgl.)** | **2** | **3** |
|---|---|---|---|---|
| **A-1** | [Gew.-%] | - | 98,6 | 97,6 |
| **A-2** | [Gew.-%] | 59,35 | - | - |
| **A-3** | [Gew.-%] | 40,0 | - | - |
| **B*-1** | [Gew.-%] | - | 1,0 | 2,0 |
| **C-1** | [Gew.-%] | - | 0,4 | 0,4 |
| **C-2** | [Gew.-%] | 0,65 | - | - |

| **Chemikalienbeständigkeit (ESC) bei 1.4% Rfd.** | | | | |
|---|---|---|---|---|
| Oberflächenbeschaffenheit nach 1 Tag Lagerung | | kV | kV | kV |
| Oberflächenbeschaffenheit nach 4 Tagen Lagerung | | A1 | kV | kV |

Aus Tabelle 1 ist ersichtlich, dass nur die erfindungsgemäße Zusammensetzungen gemäß den Beispielen 2 und 3 die zugrundeliegende Aufgabe lösen, nicht jedoch die Zusammensetzung gemäß dem Vergleichsbeispiel 1, bei dem es sich um ein herkömmliches Polycarbonat mit insgesamt hohem Molekulargewicht und hoher Chemikalienbeständigkeit handelt, d.h. nur die erfindungsgemäßen Zusammensetzungen gemäß den Beispielen 2 und 3 weisen eine gute Intralipidbeständigkeit auch bei längerer Belastung auf und zeigen somit keine Veränderung an der Oberfläche.

## Patentansprüche

1. Medizintechnisches Produkt oder Teil eines medizintechnischen Produktes,
wobei das medizintechnische Produkt oder Teil eines medizintechnischen Produktes ein Element, bestehend aus einer thermoplastischen Zusammensetzung, welche die folgenden Komponenten enthält, umfasst:
A) mindestens 75 Gew.-% aromatisches Polycarbonat und
B) 0,2 bis 1,5 Gew.-% funktionalisiertes Siloxan-Polymer, das nur aus Siloxan-Einheiten als Monomereinheiten aufgebaut ist, wobei sich die Mengenangaben auf das Gesamtgewicht der thermoplastischen Zusammensetzung beziehen und
wobei das Element dazu bestimmt ist, während der bestimmungsgemäßen Verwendung des medizintechnischen Produktes in Kontakt mit Intralipid zu stehen.

2. Medizintechnisches Produkt oder Teil eines medizintechnischen Produktes nach Anspruch 1, wobei das medizintechnische Produkt für die Verwendung in der parenteralen Ernährung bestimmt ist.

3. Medizintechnisches Produkt oder Teil eines medizintechnischen Produktes nach Anspruch 1 oder 2, wobei das medizintechnische Produkt oder Teil eines medizintechnischen Produktes ein Schlauchverbinder, ein Dreiwegehahn, ein Manifold, eine Tropfkammer, ein Luer-Konnektor, ein IV-Katheter ist.

4. Medizintechnisches Produkt oder Teil eines medizintechnischen Produktes nach einem der vorhergehenden Ansprüche, wobei das aromatische Polycarbonat Bisphenol A-basiertes Homopolycarbonat ist.

5. Medizintechnisches Produkt oder Teil eines medizintechnischen Produktes nach einem der vorhergehenden Ansprüche, wobei das aromatische Polycarbonat eine Schmelzevolumenfließrate MVR von 15 bis 35 cm³/(10 min), bestimmt nach ISO 1133:2012-03 bei einer Prüftemperatur 300°C und 1,2 kg Belastung, aufweist.

6. Medizintechnisches Produkt oder Teil eines medizintechnischen Produktes nach einem der vorhergehenden Ansprüche, wobei die thermoplastische Zusammensetzung aus den folgenden Komponenten besteht:
A) mindestens 90 Gew.-% aromatischem Polycarbonat,
B) 0,2 bis 1,5 Gew.-% funktionalisiertem Siloxan-Polymer, das nur aus Siloxan-Einheiten als Monomereinheiten aufgebaut ist,
und
C) optional einem oder mehreren weiteren Additiven, ausgewählt aus der Gruppe, bestehend aus Thermostabilisatoren, Flammschutzmitteln, Antioxidantien, Bestrahlungsstabilisatoren, Entformungsmitteln, Antitropfmitteln, UV-Absorbern, IR-Absorbern, Schlagzähmodifikatoren, optischen Aufhellern, Füllstoffen, Lichtstreumitteln, Hydrolysestabilisatoren, Umesterungsstabilisatoren, Verträglichkeitsvermittlern, organischen Farbmitteln, organischen Pigmenten, anorganischen Pigmenten und/oder Additiven zur Lasermarkierung,
wobei sich die Mengenangaben auf das Gesamtgewicht der thermoplastischen Zusammensetzung beziehen.

7. Medizintechnisches Produkt oder Teil eines medizintechnischen Produktes, wobei die thermoplastische Zusammensetzung mindestens 95 Gew.-% aromatisches Polycarbonat enthält.

8. Medizintechnisches Produkt oder Teil eines medizintechnischen Produktes nach einem der vorhergehenden Ansprüche, wobei die thermoplastische Zusammensetzung aus den folgenden Komponenten besteht:
A) mindestens 95 Gew.-% aromatischem Polycarbonat,
B) 0,2 bis 1,5 Gew.-% funktionalisiertem Siloxan-Polymer, das nur aus Siloxan-Einheiten als Monomereinheiten aufgebaut ist,
und
C) optional einem oder mehreren weiteren Additiven, ausgewählt aus der Gruppe, bestehend aus Entformungsmitteln, Thermostabilisatoren, Antioxidantien, Farbmitteln, Pigmenten.

9. Medizintechnisches Produkt oder Teil eines medizintechnischen Produktes nach einem der vorhergehenden Ansprüche, wobei 0,5 bis 1 Gew.-% an Komponente B eingesetzt werden.

10. Medizintechnisches Produkt oder Teil eines medizintechnischen Produktes nach einem der vorhergehenden Ansprüche, wobei das aromatische Polycarbonat eine Schmelzevolumenfließrate von 16 bis 20 cm³/(10 min), bestimmt nach ISO 1133:2012-03 bei einer Prüftemperatur 300°C und 1,2 kg Belastung, aufweist.

11. Medizintechnisches Produkt oder Teil eines medizintechnischen Produktes nach einem der vorhergehenden Ansprüche, wobei das funktionalisierte Siloxan-Polymer ein gewichtsmittleres Molekulargewicht M_{w}, bestimmt mittels GPC in Tetrahydrofuran (THF), kalibriert gegen Polystyrol-Standards, von > 500 000 g/mol aufweist.

12. Medizintechnisches Produkt oder Teil eines medizintechnischen Produktes nach einem der vorhergehenden Ansprüche, wobei das funktionalisierte Siloxan-Polymer Dimethylsiloxan-Einheiten aufweist.

13. Medizintechnisches Produkt oder Teil eines medizintechnischen Produktes nach einem der vorhergehenden Ansprüche, wobei das funktionalisierte Siloxan-Polymer Hydroxylgruppen aufweist.

14. Verwendung von funktionalisiertem Siloxan-Polymer zur Verbesserung der Intralipidbeständigkeit von auf aromatischem Polycarbonat basierenden Zusammensetzungen.

15. Verwendung nach Anspruch 14, wobei das funktionalisierte Siloxan-Polymer ein gewichtsmittleres Molekulargewicht M_{w}, bestimmt mittels GPC in Tetrahydrofuran (THF), kalibriert gegen Polystyrol-Standards, von > 500 000 g/mol aufweist, Dimethylsiloxan-Einheiten und Hydroxylgruppen aufweist.
